# EUROPEAN PATENT APPLICATION

(11) **EP 3 037 996 A2**
(43) Date of publication of application: **29.06.2016**
(21) Application number: 15002078.2
(22) Date of filing: 14.07.2015
(51) Int. Cl.: G06F 19/00

(54) **TELEMEDICAL METHOD AND SYSTEM FOR PATIENT MONITORING**

(30) Priority: 15.07.2014 PL 40886014
(71) Applicant: Moneo Pharma Group Sp. z o.o., 01-015 Warszawa (PL)
(72) Inventor: Krzystanek, Marek, 40-553 Katowice (PL); Wieczorek, Michal, 02-972 Warszawa (PL); Czerwinski, Mariusz, 62-400 Slupca (PL)
(74) Representative: Kondrat, Mariusz

(57) **Abstract**

Invention describes telemedical method for patient monitoring comprising following steps: monitoring of patient drug intake, controlling of patient health status and disease prevention, conducting of patient-physician physician-patient video conversations, contact between physician and patient's caregiver, updating of pharmacological therapy plan after performed video conversation, synchronization of pharmacological treatment plan with treatment plan on patient mobile device, database maintenance for all medical occurrences during treatment, especially visits, interviews, prescribed medicines, confirmations of medicine intake, shearing of educational materials in particular video library and educational audiobooks, monitoring of adverse events, remote execution of therapeutic recommendations. Invention comprises also telemedical system for patient monitoring.

## Description

Subject of the invention is telemedical method and system for patient monitoring, especially during psychiatric, hypertension, allergological (in case of asthma), pulmonary, urologic therapy or in case of vaccinations. Patient monitoring is based on monitoring of his health status as well as educational activities.

Main cause of low effectiveness of chronic diseases therapy in Poland and around the world is limited access of patient to doctor. Possibility of consultation with a doctor is limited for patient by queues in health clinics and doctor's consulting rooms. Practically, waiting time for admission or for control visit lasts on average 1 to 3 months. For example in case of ineffective dose of given drug or appearing of side effects patient practically must rely on flier attached to the drug, on which hundreds of potential adverse effects are enlisted. Without consultation with a doctor, listening to his comments to information given in the flyer and without professional advice, most of the patients stop to take medications and discontinue treatment. Efficient communication between patient and doctor can prevent discontinuation in medication intake in such a scenario. Premature discontinuation of medication intake is one of the most common cause of the treatment inefficiency, recurrence or worsening of illness symptoms and loosing of possibility for full family, social and professional functioning.

In schizophrenia after 2-3 months of treatment on average 60% of patients stop treatment without consulting a doctor and even 80% after 2 years (Kemmler G, Hummer M, Widschwendter C, Fleischhacker WW. Dropoutrates in controlled and active-control clinical trials of antipsychotic drugs: a meta-analysis; Arch Gen Psychiatry 2005, 62, 1305-1312). In long-term, 1.5 year long study concerning effectiveness of CATIE schizophrenia treatment 74% of patients did not finish treatment and resigned before 1 year (Lieberman JA, Stroup TS, McEvoy JP, Swartz MS, Rosenheck RA, Perkins DO, Keefe RS, Davis SM, Davis CE, Lebowitz BD, Severe J, Hsiao JK; Clinical Antipsychotic Trials of Intervention Effectiveness (CATIE) Investigators. Effectiveness of antipsychotic drugs in patients with chronic schizophrenia. N Engl J Med 2005, 353, 1209-1223). In one year study with first-time schizophrenia EUFEST episode patients, on average 30% of the patients did not finish the study (Kahn RS, Fleischhacker WW, Boter H, Davidson M, Vergouwe Y, Keet IP, Gheorghe MD, Rybakowski JK, Galderisi S, Libiger J, Hummer M, Dollfus S, López-Ibor JJ, Hranov LG, Gaebel W, Peuskens J, Lindefors N, Riecher-Rössler A, Grobbee DE; EUFEST study group. Effectiveness of antipsychotic drugs in first-episode schizophrenia and schizophreniform disorder: an open randomized clinical trial. Lancet. 2008, 371, 1085-1097). Possibility to consult health status, treatment effectiveness and its tolerance in the critical day, when patient stops treatment, can change this situation.

Appropriate long-term treatment is an essential condition of good long-term prognosis concerning quality of life and lack of psychical disorder recurrences. For example, it is estimated that schizophrenic disorders should be treated according to episode - fist-time must be treated at least 2 years and each recurrence 5 years, in depression treatment time must last at least 6 months, and 1 year in case of anxiety disorders.

In case of patients with arterial blood hypertension it is crucial to measure blood pressure on regular basis, as well as to take care about proper diet and provide adequate amount of exercise.

Patient drug treatment with identified pulmonary diseases requires constant drug intake e.g. inhalable one or controlling of level of some allergens in the air.

Children and especially school-age children are vulnerable to decreased immunity against certain diseases as a result of lack of recommended booster doses intake of recommended preventive vaccines.

Men above age 50 are at risk of prostate cancer.

In all areas of health, long-term and regular appliance to doctor recommendation, along with measuring of arterial blood pressure, measuring of Prostate Specific Antigen (PSA) level, controlling of pollination calendar or intake of other recommended doses of preventive vaccines, especially those, which are divided into smaller doses spread on longer time, is the factor ensuring efficient therapy.

Telemedicine gives an improvement in access to medical services. It is based on the use of electronic information and modern communication technologies in order to provide remote medical care (Angaran DM. Telemedicine and telepharmacy: current status and future implications. Am J HealthSyst Pharm 1999, 56, 1405-1426, WO0070529, US2006064319, PL359740). Electronic health care (e-health) provides education related to diseases and their prevention, enables diagnostic process and treatment and in some countries even medicine prescription (Angaran DM. Telemedicine and telepharmacy: current status and future implications. Am J HealthSyst Pharm 1999, 56, 1405-1426).

From WO9904043A1 application there is robust and precise system known, which can be used to detect many types of infectious diseases, chronic diseases, genetic diseases, nutritional, environmental deficiencies and general health problems, fertility problems, mental disorders, drug abuse, allergies etc., as well as to provide non-invasive tests such as automatic vision tests, hearing tests and testing of cognitive functions in order to monitor Alzheimer's disease progression.

There is known use of medical platform in order to identify and treat mental disorders (US2006052674, BRPI0706011). Method from US2006052674 description comprises steps of assessment of the patients' condition; evaluation and connection of potential factors contributing to the decrease in physical condition; diagnosis of the decrease. This makes it possible to ensure a proper management of drugs and medical factors and beginning of treatment with psychotropic substances.

BRPI0706011 application relates to monitoring method of patient with mental disorders, especially with mood disorders, in particular through the network and mobile communication, what enables to measure their psychological states in their responses to questionnaires sent remotely using mobile devices.

Approaches to apply telepsychiatry using internet were already conducted in Poland, however internet hints in form of e-mail message cannot be an equivalent of visit made in reality (Krzystanek M, Krupka-Matuszczyk I. Telepsychiatria - internetowe porady psychiatryczne. Psychiatr. Pol. 2003, 37, 1073-1082). Especially in psychiatry facial expression and body language (pantomimic), which correspond with information communicated in verbal way, are crucial. Observation of a patient during psychiatric examination and not just verbal message are necessary for a reliable assessment of his mental status. Without possibility for visual contact with the patient telepsychiatry is limited only to the responses on messages sent to a physician from the patient, where doctor can only suggest very cautiously and using general terms how to proceed further (Krzystanek M, Krupka-Matuszczyk I. Telepsychiatria - internetowe porady psychiatryczne. Psychiatr. Pol. 2003, 37, 1073-1082; Terry NP: In most of cases these suggestions come to the point of meeting and personal conversation with psychiatrist.

Present situation in the medical services market in psychiatry in Poland restrict access of health service provider - psychiatrists - to the patients. This causes measurable adverse effect on patients in the form of irregular compliance with pharmacological recommendations, discontinuation of therapy, disease recurrence and deterioration in long-term prognosis.

Existing solutions do not provide a comprehensive solution which would manage to efficiently lead and supervise patient therapy. Only combining of traditional way in which patient interact with doctor with system, which allows for remote contact between doctor and patient or his caregiver, to control drug intake, to examine using clinical scales, education, to update and synchronize pharmacological therapy plan and to monitor adverse events, to perform database maintenance of all medical occurrences during treatment, especially visits, interviews, prescribed medicines, to confirm drug intake, gives measurable, positive results. Such a combination of few functionalities in one system facilitates faster and more effective achievement of stable health condition till full recovery by a patient suffering from mental illnesses.

The purpose of the invention is to provide an effective telemedical system for long-term constant support of intensive care, especially psychiatric, allergological, pulmonary, urologic hypertension treatment or preventive vaccinations by constant control of physician over treatment course in form of televisits initiated by a physician or patient or by notifications about drug intake, registration of appointments in consulting room, eventual change of pharmacological therapy plan, on-line education, education in the form of video- and audiobooks, clinical scale test examination, monitoring of adverse events, conducting of cognitive functions training in psychiatric treatment, constant monitoring of allergen and environmental factors influence (humidity, temperature), on frequency of temporary use of emergency drugs e.g. in asthma therapy, remote monitoring of blood pressure measurement results and maintenance of database for all medical occurrences during treatment.

Solution according to invention is addressed to i.a. the group of schizophrenia patients. Schizophrenia is a disease, from which suffers approximately 1% of the population. Modern neuroleptic drugs used for schizophrenia therapy enable treatment of the disease symptoms, but treatment success depends on the designation of the neuroleptic effective dose, proper treatment tolerance, regular drug intake and sufficiently long treatment stabilization phase.

The essence of the invention is telemedical method for patient monitoring, characterized by consisting of following steps:
a) monitoring of medicine intake by patient
b) controlling of patient health and disease prevention
c) conducting of patient-physician and physician-patient video calls
d) contacting of the physician with patient caregiver
e) update of pharmacological therapy plan after conducted video call
f) synchronization of treatment plan with treatment plan in patient mobile device
g) database maintenance for all medical occurrences during treatment, especially visits, interviews, prescribed medicines, conversations of medicine intake.
h) sharing of educational materials in particular video library and educational audiobooks.
i) monitoring of adverse events
j) remote execution of therapeutic recommendations

Preferably, a) step of telemedical method is based on:
- informing patient about pharmacological therapy plan
- informing patient about a need to take each dose of medication
- recording of confirmation concerning drug dose intake
- processing of confirmation to the graphical report form for the doctor
- preparing of patient cooperation analysis concerning drug intake during time of treatment

Preferably, b) step of telemedical method is based on:
- examinations using clinical scales, and/or
- remote monitoring of clinical parameters, and/or
- conducting of geolocation and environmental risks correlation

Preferably, clinical scale examination is conducted in real-time, wherein examination results are processed to the form of graphical report, and moreover data analysis is performed from clinical scales examination during time of treatment.

Preferably, remote monitoring of clinical parameters is conducted directly by transfer of the measurement results from measuring device to the module or indirectly by input of measurement results to the module by patient.

Preferably, remote monitoring of clinical parameters is based on:
- recording of arterial blood pressure measurement, and/or
- recording of blood saturation measurement, or
- recording of pulse measurement, or
- recording of level of laboratory results e.g.: PSA for men, and/or
- recording of vaccination dose intake

Preferably, recording of arterial blood pressure, pulse and blood saturation (level of haemoglobin saturation with oxygen) is based on measurement of arterial blood pressure and pulse using a device for measuring of blood pressure, heart rate using pulse oximeter, which are remotely connected to video-communicator.

Preferably, recording of PSA level measurement results to the system is performed by a patient. Preferably, recording of vaccination dose intake is performed after intake of next vaccination dose. Preferably, generation of geolocalizing and environmental risks correlation is based on determination of patient localization and allergen concentration in the vicinity and sending of warning messages to the patient.

Preferably, k) step of telemedical method is based on:
- performance of cognitive trainings for patients and monitoring of changes in patients cognitive performance during treatment, or
- recommending to conduct blood pressure measurement at home, or
- recommending to conduct PSA level measurement in the laboratory.

Preferably, cognitive training is based on:
- training of cognitive functions
- measuring of cognitive functions
- normalization of cognitive functions
- supervision of patients performance during cognitive trainings,
- recording of cognitive functions parameters
- generation of graphic reports
- analysis of those parameters during time of therapy

Preferably, telemedical method inform patient about visit in medical practice/consulting room, enables patient registration for a visit in medical practice/consulting room and generates medical documentation in electronic format.

The subject of the invention is also telemedical system for patient monitoring, characterized by consisting of following modules:
a) module for monitoring of drug intake by a patient (008)
b) module for controlling of patient health status and disease prevention (004)
c) module for conducting of physician-patient and patient-physician video conversations and for contacting a physician with patient caregiver (002)
d) module used for updating of pharmacological therapy plan after conducted video call (005)
e) module for synchronizing pharmacological therapy plan and therapeutic recommendations with therapy plan in patient mobile device (013)
f) module used for database maintenance for all medical occurrences during treatment, especially visits, interviews, prescribed medicines, confirmations of medicine intake (003)
g) module used for sharing of educational materials in particular video library and educational audiobooks (018)
h) module used to monitor adverse events (017)
i) module used for remote conduction of therapeutical recommendation (012).

Preferably, module described in point a) of telemedical system:
- informs patient about pharmacological therapy plan
- informs patient about a need to take each dose of medication
- records confirmation concerning drug dose intake,
- processes confirmations to the graphical report form for the doctor,
- performs patient cooperation analysis concerning drug intake during time of treatment

Preferably, module described in point b) of telemedical system consist of modules for:
- examination using clinical scales, and/or
- remote monitoring of clinical parameters, and/or
- conducting of geolocation and environmental risks correlation

Preferably, clinical scale examination is conducted in real-time, wherein examination results are processed to the form of graphical report, and moreover data analysis is performed from clinical scales examination during time of treatment.

Preferably, remote monitoring of clinical parameters is conducted directly by transfer of the measurement results from measuring device to the module or indirectly by input of measurement results to the module by patient.

Preferably, module for remote monitoring of clinical parameters includes modules:
- for recording of arterial blood pressure, and/or
- for recording of blood saturation measurements, or
- for recording of pulse measurement, or
- for recording of level of laboratory results e.g.: PSA for men, and/or
- for recording of vaccination dose intake

Preferably, module for recording of arterial blood pressure, blood saturation and pulse transfers results of arterial blood pressure measurement, blood saturation and pulse through device for measurement of arterial blood pressure, which is remotely connected to video communicator. Preferably, module for recording of PSA measurement level results in the system saves and stores results entered by a patient

Preferably, module for recording of vaccination dose intake is performed after intake of next vaccination dose.

Preferably, module for generation of geolocation and environmental risks correlation performs determination of patient localization and allergen concentration in his vicinity and sending of warning messages to the patient.

Preferably, module described in point b) of telemedical system is a video communicator:
Preferably, module described in point d) of telemedical system performs updates of pharmacological treatment plan by input of data concerning modifications of pharmacological treatment plan, records it and transfers to module e).

Preferably, module described in point e) of telemedical system transfers data recorded in module d) to patient mobile device, wherein message appears on the patient mobile device concerning change in pharmacological treatment plan.

Preferably, module described in paragraph (f) of telemedical system collects data about all medical occurrences during treatment obtained both from the patient and physician, wherein these data are also provided in form of reports, in tabular and graph forms (004) (014).

Preferably, module described in paragraph (g) of telemedical system collects educational material, especially audio- and videobooks in available formats and shares them by internet, wherein physician recommends to open educational materials and sends given educational material to the patient. Preferably, module described in h) paragraph of telemedical system collects information about adverse events, records them and stores on the clinical server.

Preferably, module described in point i) of telemedical system consist of modules:
- for performance of cognitive trainings for patients and monitoring of changes in patients cognitive performance during treatment, and/or
- for recommending to conduct blood pressure measurement at home, and/or
- for recommending to perform rehabilitation, and/or
- for recommending to conduct PSA level measurement in the laboratory.

Preferably, module for conduction of cognitive trainings conducts cognitive trainings, which consist of:
- training of cognitive functions
- measuring of cognitive functions
- normalization of cognitive functions
- supervision of patients performance during cognitive trainings,
- recording of cognitive functions parameters
- generation of graphic reports
- analysis of those parameters during time of therapy

Preferably, telemedical system informs patient about visit in medical practice/consulting room, enables patient registration for a visit in medical practice/consulting room and generates medical documentation in electronic format.

Main reason for ineffective treatment in psychiatry, especially for schizophrenia, is inefficient cooperation of patient during treatment, lack of compliance to physician recommendation concerning drug intake, irregular drug intake and/or arbitrary discontinuation of treatment. It allows patient to contact the physician in any moment, when he needs a conversation or information concerning treatment or side effects symptoms of drugs intake, conducting of successful psychoeducation may prevent discontinuation of medication intake and deterioration of patient health status. One of the system functionality according to the invention is also monitoring of therapeutic plan with implemented module which reminds patient about time for drug intake or about necessity of planned training execution, video visit or appointment in clinic or in doctor's consultation room. Feedback coming from patient gives a possibility to monitor therapy progress such as e.g. drug intake. It is the form of monitoring of patient cooperation in treatment and compliance with the physician prescriptions.

In the modern world institutional psychiatry is going to be substituted by environmental therapy. Its main goal is to treat patients in their life environment, most optimally at home, without separation from family context, friends and their place of living. On the one hand it reduces stress associated with hospital treatment and on the other enables the use of resources located in patient environment (family, friends) who can help in overcoming of patient mental crisis. Telemedical method and system for patient monitoring gives the possibility to cure patient in his place of residence.

Telemedical system enables simultaneous reduction of patient treatment costs outside hospital for example: psychiatric. In case of psychiatric examination, examination includes patient interview, observation of behaviour, reaction, facial expressions and pantomimic. Only on the first visit it is mandatory to perform physical patient examination, while during the successive visits reported physical discomfort are crucial during decision about need for a physical examination. Telemedical services system is a high-value equivalent of the visit in consulting room or in psychiatric clinic. In case of need for physical examination or receiving of prescription for a drug, physician can make an appointment for visit in medical practice or in consulting room for a patient through telecommunicator (003).

Telemedical system is easy to widespread and can be used everywhere by anyone.

Video conversation module (002) can be used practically through telemedical method and patient monitoring system, has a practical value for a physician in his profession. Allows him to conduct medical documentation, monitoring of patient adverse events as well as his mental state and effectiveness of remote therapy.

Telemedical method gives a possibility to a physician e.g. psychiatrist, for a constant control of patient mental state, constant control of recommended treatment efficiency (004), constant modification of therapy (005) in case of occurrence of major premises, effective carrying out of necessary psychoeducation, control over regularity of medicines intake (004), appointment schedule. Telemedical method enables conduction of cognitive function training in form of cognitive trainings implemented into the system, which are performed by a patient two times a week on his own. During the workouts parameters of cognitive functions are also measured, what gives to the physician a possibility to monitor progress of the treatment.

Telemedical method and system enables remote control of arterial blood pressure by patients under constant supervision by physicians, eliminating so called white coat syndrome.

The system provides full monitoring and gives a possibility to associate data, that describe arterial blood pressure, drugs intake (patient compliance). That leads to intake of prescribed medicines in the appropriate doses and time of the day and introduction of recommended changes in lifestyle and physical activity, what ensures stable and wide estimation of health status and correlation between drug usage and patient clinical status on-line.

Telemedical method is a system which enables ergonomic recording of drug compliance of patients with asthma and COPD (chronic obstructive pulmonary disease). The system provides also continuous monitoring of allergens and environmental factors (such as humidity, temperature), on frequency of drugs usage, including temporary use of emergency drugs i.e.: beta-mimetics (e.g. SABA) or steroids and continuous access to educational materials.

Telemedical system is connected with an inhaler in order to monitor patient compliance with their clinical status and providing an access to the educational materials for the patients concerning prevention and treatment of asthma and COPD.

Drug compliance data are supplemented with environmental data: time of day, estimated localization, atmospheric pressure, air humidity and allergens present (correlation with on-line pollinating calendar: patient localization and allergen concentration).

Telemedical method and system also enable knowledge dissemination concerning vaccines, reminding about vaccination dates and full assessment of vaccination safety. System covers the nurses network and two groups of patients: parents/caregivers of the children and group of adults. System is also intended to remind patients about dates of compulsory and non-compulsory vaccinations, give information about disease entities for which vaccination is recommended (educational function) and to allow for remote security assessment.

System provides supervision of patient health status assessment before vaccination, what is a key element in the process of vaccination, vaccination follow up process (reminding of next dose of vaccine or vaccination from children vaccination schedule) for patient, nurse and physician.

System is also an educational base rising patient awareness to the legitimacy of vaccinations, what have a crucial influence on the level of vaccinations in society.

Telemedical method and system is used to conduct on-line process of prostate cancer prevention in group of men older than 50 years.

System can be used to assess risk of prostate cancer among other things by remote recommendation of clinical scale tests such as e.g.: IPPS, possibility of data input from laboratory tests - PSA level, system manages the diagnosis program for patients introduced to the system depending on risk group, in order to minimise the risk of too late prostate cancer detection. System constantly monitors patient drug compliance and displays patient risk factor (RF) coming from cyclically carried out clinical scale test and entered results of laboratory tests (PSA).

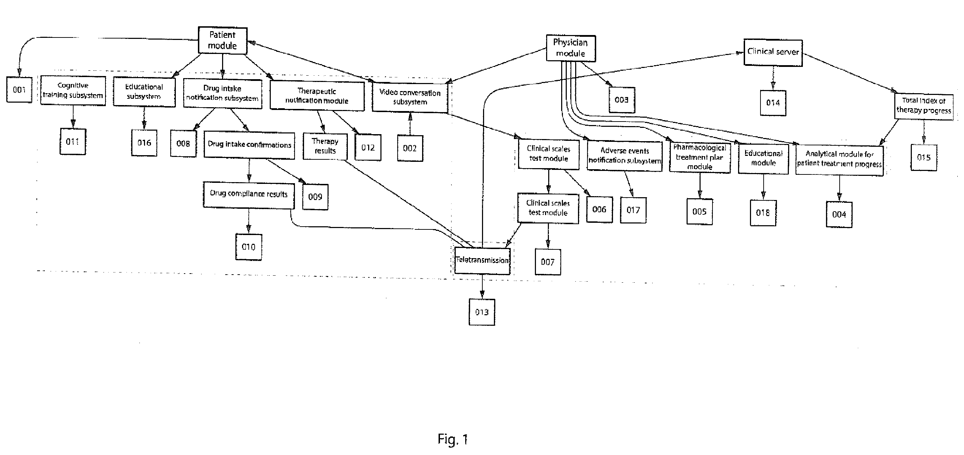

Telemedical method of patient monitoring is shown on figure, where fig. 1 shows block scheme of the mode of action for the invention and fig. 2 shows scheme of library base operation, fig. 3 shows results of the first phase of cognitive training, fig. 4 shows results of the second phase of cognitive training, fig. 5 shows results of the third phase of cognitive training, fig. 6 shows results of the patient drug compliance, fig. 7. shows efficacy of reminder display concerning necessity of drug intake. Telemedical system according to invention is working in the IP network and/or SMS, wherein peripheral devices (e.g. blood pressure meter) is connected with other devices (e.g. Server) by e.g. Bluetooth. According to the invention telemedical method of patient monitoring utilizes telemedical system and is based on the fact, that patient receives message by mobile device (smartphone, tablet) about intake of drug dose prescribed by a physician. Subsequently, patient confirms drug dose intake (008). Patient drug intake monitoring module records and stores all possible data concerning the patient drug dose intake and enables generation of reports in form of tables or graphs (004).

Physician can be up to date, by his mobile device (smartphone, tablets) (003) has the ability to monitor the process of patient drug intake and contacts patients using module for patient-physician and physician-patient video chatting if necessary.

According to the therapy plan and recommendations of treating physician, physician conducts remote clinical scale test (006), i.e. conducts test using psychometric scale e.g.: Calgary, CGI, PANSS in case of psychiatry. Module used for examinations using clinic scales e.g.: IPSS for urology, enables conducting of this measurement in other medical specialities, as well as records and analyses its result. Based on that physician can update pharmacological treatment plan (005).

In every moment of therapy physician can contact patient caregiver in order to complement an examination interview and in order to provide information about patient health status.

Telemedical method enables using of audio and videobook library concerning schizophrenia, its treatment and associated problems, which are available on the Internet and used in practice. Telemedical treatment method enables both training of patient cognitive functions and also measuring of their parameters and monitoring of cognitive function changes during therapy. Telemedical system according to the invention comprises following modules:
Module for monitoring of patient drugs intake (005) on the one hand facilitates regular drug intake by patient and also enables control over process by a physician. High compliance, i.e., in a broad context, good cooperation between a physician and patient, is in practice predicted effect in form of higher long-term prognosis for schizophrenia therapy.
Module used to control patient health status and health prevention contains modules used for examination with clinical scales, remote monitoring of clinical parameters, geolocation and environmental risks correlation.
Module used for clinical scales examination (006) enables physician to perform examination using one of the chosen scales: e.g.: psychometric (PANSS, Calgary, CGI) and subsequently transfers those data to the module of management database of all medical occurrences.

For example clinical scales broadly used for examinations, which can be used for the assessment of patient:

### PANSS scale:

Assesses 30 schizophrenia symptoms including:
16 general psychopathological symptoms.
7 positive symptoms
7 negative symptoms
16 general psychopathological symptoms,

It is filled by a physician, each symptom is evaluated in a 1-7 point scale. Therefore, overall scale result may range between 30 and 210 points. Results of the questions about positive and negative symptoms are often given separately, that may range between 7 and 49 points.

### Calgary scale:

Scale used to assess the level of depression in schizophrenia. It consists of 9 questions. It is filled by a physician, each symptom is evaluated in a 0-3 point scale. Therefore, overall scale result may range between 0 and 27 points.
IPSS scale - is used in order to assess intensification of complaint associated with urination disorders in course of prostate diseases. Test results can assist to assess patient clinical state and to decide on further therapeutic treatment. Scale consist of 8 questions assessed in 6 points scale,

Module for remote monitoring of clinical parameters contains modules and/or modules used to monitor different clinical parameters based on data transferred directly to the module from patient measuring device or entered by a patient after conduction of analysis in laboratory. This module includes in particular the modules:
- recording of arterial blood pressure, and/or
- recording of blood saturation measurements, and/or
- recording of pulse measurement, and/or
- recording of level of laboratory results e.g.: PSA for men, and/or
- for recording of vaccination dose intake
- recording of clinical studies results.

### Model for geolocation and environmental risks correlation

System according to the invention shows the effects of allergens on increase in amount of PRN inhalable drugs intake in case of increase in allergen concentration (geolocation connected with updated on-line pollination calendar). Physician has constant access to maintenance intake frequency of inhalable drugs (according to sent treatment plan to the patient) and ability to modify the drug dose in a situation requiring the modification.

Module for conducting of physician-patient and patient-physician video conversations and for contacting a physician with patient caregiver (002) Conducting of televisit using telemedical system is practical and easy thanks to software which is compatible with video-messengers. Video communicators enables performance of video connection, which was conducted through packet network (Internet) using WiFi or GSM connection.

Module used to update pharmacological therapy plan (005): After conducted video conversation it enables further induction of changes in taken drugs, what is based on introduction of new drugs, change in existing one, change in dosing type, establishment of proper drug dose. Patient receives a message about treatment plan modification.

Module used to synchronize pharmacological treatment plan (013) with therapy plan in a patient mobile device transfers information concerning modification of drug treatment plan performed by a physician and treatment recommendations to patient device.

Module used for database maintenance for all medical occurrences (014) during treatment, especially visits, interviews, prescribed medicines, confirmations of medicine intake. Database of medical occurrences, which are available for the physician, patient and on the clinical server, is generated in parallel using this module,.

Module used for sharing of educational materials (16) in particular video library and educational audiobooks.

Library of educational materials including audio- and videobooks concerning schizophrenia, contains a database, which goal is to collect and share collected material, both text and multimedia one. Audiovisual collections are stored in form converted to recommended formats providing desired degree of message quality (image and sound clarity etc.) and additional functionalities (tables of content, references etc.).

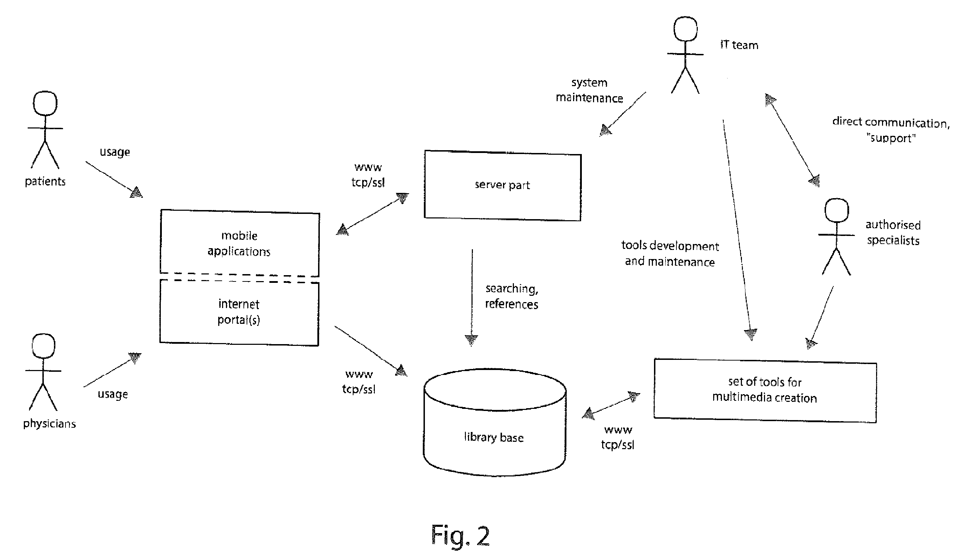

Library base is located as a central point of the telemedical system (Fig. 2).

### Module used to monitor adverse events (017)

An adverse event of medical product is every unfavourable and unintended action of the medical product (according to pharmaceutical law act revised in 2013).

Module used to monitor adverse events enables control over safety of treatment by platform, which is based on the fact that:
- Patient reports in real-time an occurrence of adverse effects through a special form,
- Physician through a special form has a possibility to send this notification to the entities, which are intended to collect data on the occurrence of adverse events including:
- Responsible entity (drug manufacturer).
- The Office for Registration of Medicinal Products, Medical Devices and Biocidal Products,
- European Medicine Agency (EMA).

Module used for remote conduction of therapeutic recommendations allows the physician to conduct some actions necessary for the proper course of the therapy, e.g.: conduction of cognitive training, physical exercises, measurement of arterial blood pressure etc.

### Description of trainings:

Cognitive training - enables both measurement of patient cognitive functions and monitoring of changes in patient cognitive functions during the treatment.

RELAX TEST - before conduction of measurement using blood pressure meter, patient should fully relax and calm down, with the aim to obtain most reliable measurements. Patient should prepare comfortable condition of blood pressure measurement (secluded place, without excess of audiovisual and stress stimuli, take a position in which measurement will be performed subsequently), activate RELAX TEST function and proceed according to training recommendations.

System and telemedical method combine a lot of functionalities, which enable easy and fast communication between patient and physician during routine course of therapy, as well as in emergency cases.

Physician on his mobile device (e.g. tablet) has a possibility to input new patient data or choose patient from the list of patients, which were previously treated by him. Physician also monitors notifications concerning scale tests, televisits, visits in clinic/consulting room, waiting calls.

Physician has the ability to conduct video conversation, examination should be carried out in conditions that ensure good patient visibility and audibility. After performed examination physician answers a question, if adverse effects associated with drug treatment occurred and prepares a comment, describes a visit, writes down his observations and remarks.

Physician conducts also objective interview based on conversation with patient caregiver. System establishes a connection with the caregiver, which phone number was given by a patient during his registry as a new patient in telemedical system. This contact enables conduction of an objective interview, gathered information is used for psychometric assessment with clinical scale.

Physician has also a possibility to send a videobook, audiobook or e-book from library base to the patient. Type of sent material is dependent on doctor recommendations and its objective is to send this type of educational material, which will be most useful for the patient.

Physician can apply in the calendar day and hour of planned telemeeting. System informs him one day and one hour in advance before planned visit. In case when patient schedules his visit in consulting room, this information appears in the physician visit calendar.

Physician adjusts treatment plan depending on the results of treatment. Can change drugs or how they are administered. System transfers this information to the patient module.

Physician has the ability to prepare a prescription, which is printed out during visit of the patient in the consulting room.

Physician has the possibility to track history of events (004). These data are also required for reporting of treatment results and for indexing of telemedical services. System generates the report in form of graph concerning the call rates and their duration.

Physician has the possibility to perform clinical examination of the patient using clinical scale (006), which relies on filling of form by physician comprising questions according to the type of clinical scale.

Physician has the possibility to control patient compliance (10). In patient module it is possible to control amount of appearing notifications about drug intake in comparison with confirmed drug intakes.

Physician can also see the results of autotrainings (011) performed by the patient, define percent of correct answers, percent of incorrect answers and response time in seconds.

Physician can recommend conduction of therapeutic approach e.g. conduction of relax test training, and subsequent measurement of arterial blood pressure.

Patient has the possibility to contact the physician via his mobile device (e.g. smartphone) by activating video conversation with a physician. If physician does not answer the call, message about waiting call will appear in his system.

Patient can also observe prescribed drugs and update of the treatment plan (005). Patient can also schedule a visit in his consulting room, thanks to the possibility of physician availability verification in shared physician calendar where patient designates a day and time of his visit. Subsequently, system synchronizes the data with the calendar of the physician's visits.

Telemedical system informs patient about necessity of drug intake and confirms drug intake, planned trainings, planned visits, unsuccessful attempts of doctor - patient contact, receiving videobook or audiobook.

Patient can also perform trainings e.g. cognitive (011), wherein system reminds him about doing one training per day. Similarly patient plays video- or audiobooks.

Functioning of telemedical method and system is visualised on the exemplary patient "day": Example 1.

### Situation 1- beginning of the therapy

9.00 patient's home, drug intake and confirmation of drug intake
11.00 visit in consulting room, physician prescribes drugs and defines dosage plan.
11.25 physician sends videobook to the patient with description of therapeutic issue.
11.25 patient receives notification about receiving of new educational material
16.00 patient's home, patient performs training e.g. cognitive one
18.00 patient's home, patient receives notification about necessity of drug intake, confirms drug intake
18.10 patient's confirms drug intake
19.00 patient's home, physician calls the patient and performs video conversation with examination e.g. using clinical scale, results are simultaneously recorded in patient inventory.
20.15 patient's home, patient watches videobook obtained from a physician.

### Situation 2 - after few weeks of treatment

10.43 physician in his consulting room analyses treatment progress based on compliance results, cognitive trainings and scale tests, contacts using video chat, performs interview and modifies patient therapy plan.
10.50 patient's home, patient receives information about changed therapy plan
18.00 patient's home, patient receives notification about necessity of drug intake according to the new plan, confirms drug intake

### Example 2.

### Situation 1 - beginning of the therapy after few weeks during using of telemedical method and system according to the invention

8.30 patient with arterial blood hypertension performs "relax test" training
8.35 measurement of arterial blood pressure by patient
11.00 visit in doctor's consulting room, physician analyses results of arterial blood pressure and compliance from last period, physician prescribes drugs and defines plan of dosage.
11.25 physician sends videobook to the patient with description of therapeutic issue.
11.25 patient receives notification about receiving of new educational material
18.00 patient's home, patient receives notification about necessity of drug intake, confirms drug intake
19.00 patient performs "relax test" training
19.05 patient performs arterial blood pressure, blood saturation and pulse measurements
19.15 patient's home, patient watches videobook obtained from a physician

### Situation 2 - after few weeks with using of telemedical method and system according to the invention

08.30 patient performs "relax test" training
08.35 patient performs arterial blood pressure measurement - results indicate elevated blood pressure.
10.43 physician in doctor's consulting room analyses therapy progress and based on compliance results, performed "relax test" trainings and blood pressure measurements - despite good compliance, measurement results are elevated - this situation repeats for 15 days, what shows that patient is in the risk group - based on the analysis he contacts the physician using video conversation, physician performs interview and modifies therapy plan.
10.50 patient's home, patient receives information about changed therapy plan
18.00 patient's home, patient receives notification about necessity of drug intake according to the new plan, confirms drug intake
19.00 patient performs "relax test" training
19.05 measurement of arterial blood pressure by patient
19.15 patient's home, patient watches videobook obtained from a physician

In order to confirm the effectiveness of the telemedical method and system analysis of patient, responsiveness tests was performed.

Monitoring of therapy effects using telemedical method was performed using clinical scales. In this project physicians with psychological speciality were taking part.

Physician entered patient data, therapeutic recommendations concerning used drugs and planned date of the next visit in clinic or doctor's consulting room into the software. Software based on those entries was notifying patient about necessity of drug intake or planned visit date. Physician was conducting compliance monitoring based on messages transferred by patient feedback. Patient could report a need for televisit - in this case physician was connecting in real-time, and in case of temporary inaccessibility, information about patient was appearing on the list of people to contact. During video conversation these topics were discussed, where education is needed. Physician answered the questions and after video conversation sent videobooks and audiobooks connected with corresponding topics. Physician every month using video conversation was performing assessment of mental state using psychometric scale and results were recorded on his telecommunicator in section designated for a given patient. If there is a need for a meeting in clinic or in doctor's consulting room (e.g. in order to receive a prescription) visit date is appointed during video conversations. Database was developed for course and effects of treatment and with the exception of the part concerning patient personal data. During therapy database accessible for doctors and patients has been updated in regular intervals (once a month or once a quarter).

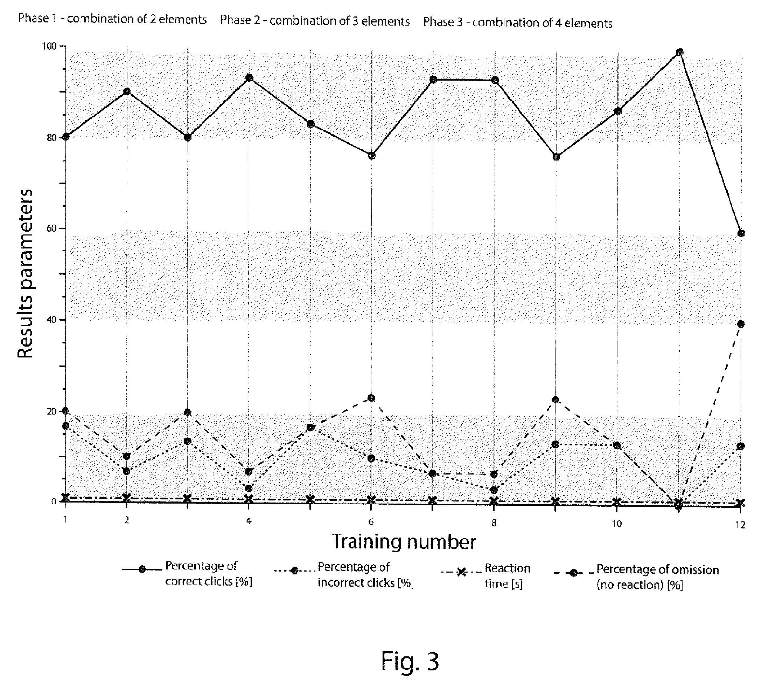

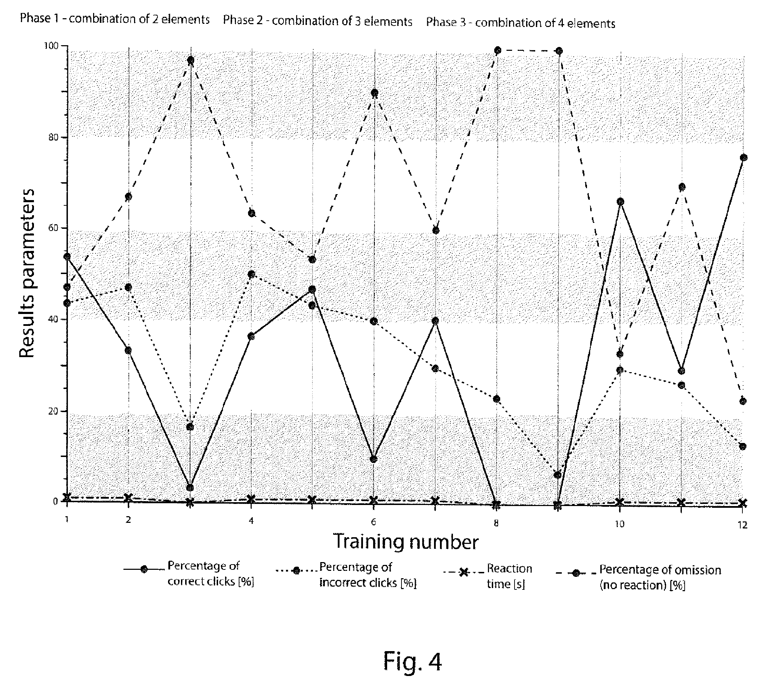

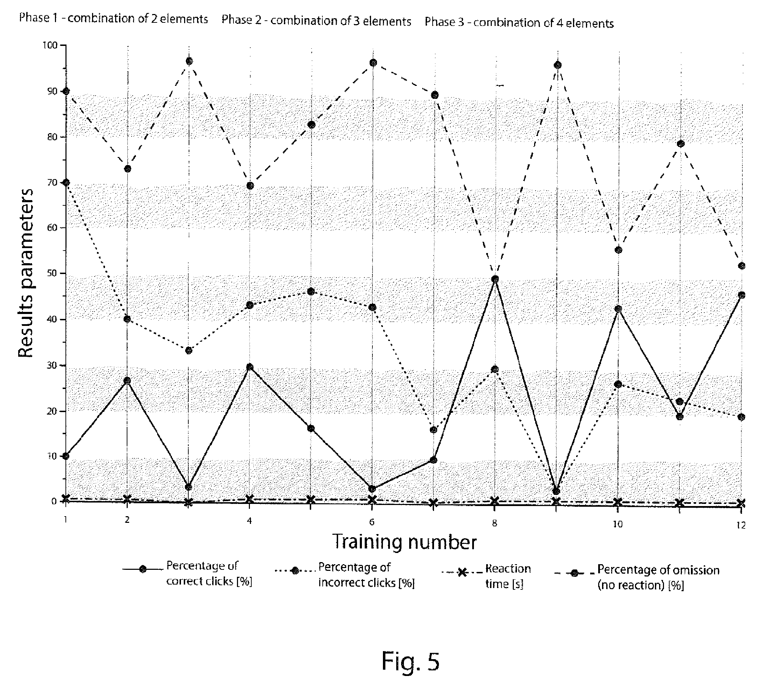

After two months of using of telemedical method according to the invention significant improvements in cognitive abilities of patient with psychological disorders has been observed, what is shown on fig. 3-5, (amount of right answers in the survey containing two elements was higher than 80%).

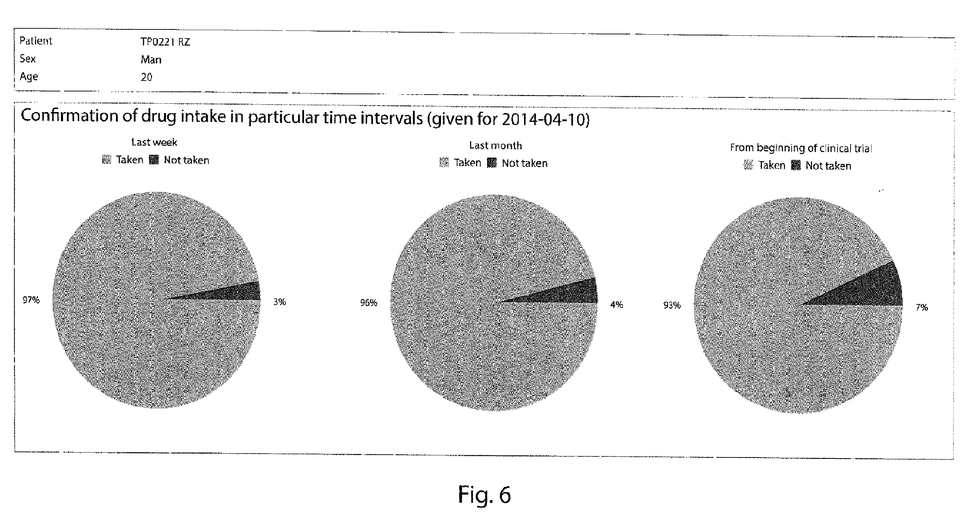

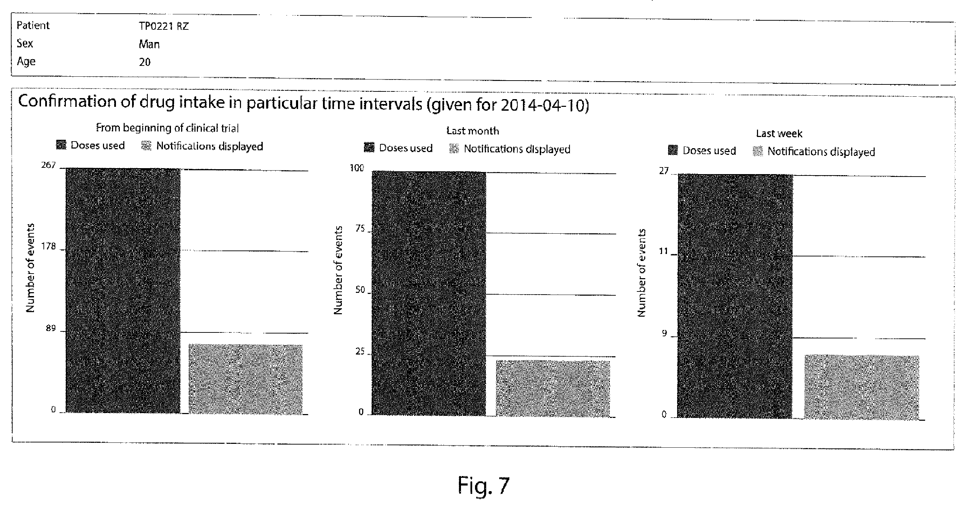

Efficiency improvement in drug intake (from 93% to 97%) has been observed as a result of notifications generated by telemedical system (fig. 6). This is confirmed by the graph shown in fig. 7, where its visible, that while using system according to the invention, patient becomes more disciplined and does not need notifications about drug intake (number of generated notifications dropped from 80% to 8%).

Patient with arterial blood hypertension has blood pressure meter at home, with which he is measuring arterial blood pressure and/or pulse. Subsequently, blood pressure meter transfers recorded result(s) directly into the system via smartphone.

System shows the correlation of appropriate pharmacotherapy and patient psychophysical activity (relax test), through correlation of results from blood pressure measurement, pulse measurement and drug compliance. Blood pressure and pulse measurements in household conditions deviate from ambulatory conditions.

Patient with asthma or chronic obstructive pulmonary disease is activating information transfer to the system (smartphone) about intake after inhalable drug administration from pressure feeder (inhaler). Smartphone is equipped with geolocation module connected with current map of allergen concentration in air. System according to the invention shows the effects of allergens on increase in amount of PRN inhalable drugs intake in case of increase in allergen concentration (geolocation connected with updated on-line pollination calendar). Physician has constant access of maintenance and pro re nata intake frequency of inhalable drugs (according to sent treatment plan to the patient) and ability to modify the drug dose in a situation requiring the modification.

Usage of system according to invention for monitoring of vaccination in patients maintains vaccination compliance consistent with vaccination calendar for children and vaccination compliance for adults, especially in case of vaccinations divided into 2 or 3 doses spread in time (e.g.: 6 months interval between the next vaccine dose).

Patient suffering from prostate cancer is making PSA antigen level test in specialized laboratory independently or after physician recommendation, and obtained results are entered to the system. System measures level of physical activity (pedometer function), what gives a possibility to control patient physical activity. Patient introduces to the system data about his age, demographics, education and previous illnesses.

System activates patient pro-health habits depending on: clinical test results e.g.: IPSS, demographics, age, education, previous illnesses, laboratory data, especially PSA level etc. Treating physician assesses patient clinical status based on clinical scale results, PSA level and drug compliance - modifies pharmacotherapy and calls the patient for unplanned verification visit.

## Claims

1. Telemedical method for patient monitoring, **characterised in that** it comprises following steps:
a) monitoring of medicine intake by patient
b) controlling of patient health status and disease prevention
c) conducting of patient-physician and physician-patient video conversations
d) contacting of the physician with patient caregiver
e) update of pharmacological therapy plan after conducted video call
f) synchronization of treatment plan with treatment plan in patient's mobile device
g) database maintenance for all medical occurrences during treatment, especially visits, interviews, prescribed medicines, confirmations of medicine intake.
h) sharing of educational materials in particular video library and educational audiobooks.
i) monitoring of adverse events
j) remote execution of therapeutic recommendations

2. Telemedical method according to claim 1, **characterised in that** the step a) is based on:
- informing patient about pharmacological therapy plan
- informing patient about a need to take each dose of medication
- recording of confirmation concerning drug dose intake
- processing of confirmation to the graphical report form for the doctor
- preparing of patient cooperation analysis concerning drug intake during time of treatment

3. Telemedical method according to claim 1, **characterised in that** the step b) is based on:
- examinations using clinical scales, and/or
- remote monitoring of clinical parameters, and/or
- conducting of geolocation and environmental risks correlation

4. Telemedical method according to claim 3, **characterised in that** the clinical scale examination is conducted in real-time, wherein examination results are processed to the form of graphical report, and moreover data analysis is performed from clinical scales examination during time of treatment.

5. Telemedical method according to claim 3, **characterised in that** the remote monitoring of clinical parameters is conducted directly by transfer of the measurement results from measuring device to the module or indirectly by input of measurement results to the module by patient.

6. Telemedical method according to claim 1 **characterised in that** the remote monitoring of clinical parameters is based on:
- recording of arterial blood pressure measurement, and/or
- recording of blood saturation measurements, or
- recording of pulse measurement, or
- recording of level of laboratory results e.g.: PSA for men, and/or
- recording of vaccination dose intake

7. Telemedical method according to claim 6, **characterised in that** the recording of arterial blood pressure, blood saturation and pulse is based on arterial blood pressure, blood saturation and pulse measurement using device for measurement of arterial blood pressure, which is remotely connected to video communicator.

8. Telemedical method according to claim 6, **characterised in that** the PSA level measurement result registration in the system is performed by a patient.

9. Telemedical method according to claim 6, **characterised in that** the recording of vaccination dose intake is performed after intake of next vaccination dose.

10. Telemedical method according to claim 1, **characterised in that** the generation of geolocation and environmental risks correlation is based on determination of patient localization and allergen concentration in the vicinity and sending of warning messages to the patient.

11. Telemedical method according to claim 1, **characterised in that** the step k) is based on:
- performance of cognitive trainings for patients and monitoring of changes in patients cognitive performance during treatment, or
- recommendations to conduct blood pressure measurement at home, or
- recommendations to conduct PSA level measurement in the laboratory.

12. Telemedical method according to claim 11, **characterised in that** the performance of cognitive trainings is based on:
- training of cognitive functions
- measuring of cognitive functions
- normalization of cognitive functions
- supervision of patients performance during cognitive trainings,
- recording of cognitive functions parameters
- generation of graphic reports
- analysis of those parameters during time of therapy

13. Telemedical method according to claim 1, **characterised in that** it informs the patient about a visit in medical practice/consulting room, enables patient registration for a visit in medical practice/consulting room and generates medical documentation in electronic format.

14. Telemedical system for patient monitoring, **characterised in that** it comprises following modules:
a) module for monitoring of drug intake by a patient (008)
b) module for controlling of patient health status and disease prevention (004)
c) module for conducting of physician-patient and patient-physician video conversations and for contacting a physician with patient caregiver (002)
d) module used for updating of pharmacological therapy plan after conducted video conversation (005)
e) module for synchronizing pharmacological therapy plan and therapeutic recommendations with therapy plan in patient's mobile device (013)
f) module used for database maintenance for all medical occurrences during treatment, especially visits, interviews, prescribed medicines, confirmations of drug intake (003)
g) module used for sharing of educational materials in particular video library and educational audiobooks (018)
h) module used to monitor adverse events (017)
i) module used for remote conduction of therapeutic recommendation (012)

15. Telemedical system according to claim 14, **characterised in that** the module described in point a):
- informs patient about pharmacological therapy plan
- informs patient about a need to take each dose of medication
- records confirmation concerning drug dose intake,
- processes confirmations to the graphical report form for the doctor,
- performs patient cooperation analysis concerning drug intake during time of treatment

16. Telemedical system according to claim 14, **characterised in that** the module described in point b): includes modules:
- for examination using clinical scales, and/or
- for remote monitoring of clinical parameters, and/or
- for conducting of geolocation and environmental risks correlation

17. Telemedical system according to claim 16, **characterised in that** the clinical scale examination is conducted in real-time, wherein examination results are processed to the form of graphical report, and moreover data analysis is performed from clinical scales examination during time of treatment.

18. Telemedical system according to claim 16, **characterised in that** the remote monitoring of clinical parameters is conducted directly by transfer of the measurement results from measuring device to the module or indirectly by input of measurement results to the module by patient.

19. Telemedical system according to claim 16, **characterised in that** the module for remote monitoring of clinical parameters includes modules:
- for recording of arterial blood pressure, or
- for recording of blood saturation measurements, or
- for recording of pulse measurement, or
- for recording of level of laboratory results e.g.: PSA for men, or
- for recording of vaccination dose intake

20. Telemedical system according to claim 19, **characterised in that** the module for recording of arterial blood pressure, blood saturation and pulse transfers results of arterial blood pressure measurement, blood saturation and pulse through device for measurement of arterial blood pressure, which is remotely connected to video communicator.

21. Telemedical system according to claim 19, **characterised in that** the module for recording of PSA measurement level results in the system saves and stores results input by a patient.

22. Telemedical system according to claim 19, **characterised in that** the module for recording of vaccination dose intake saves and stores the information about intake of next vaccination dose.

23. Telemedical system according to claim 16, **characterised in that** the module for generation of geolocation and environmental risks correlation performs determination of patient localization and allergen concentration in his vicinity and sends warning messages to the patient.

24. Telemedical system according to claim 14, **characterised in that** the module described in point c) is a videocommunicator.

25. Telemedical system according to claim 14 **characterised in that** the module described in point d),performs updates of pharmacological treatment plan by input of data concerning modifications of pharmacological treatment plan, records it and transfers to module e).

26. Telemedical system according to claim 14, **characterised in that** the module described in point e) transfers data recorded in module d) to patient's mobile device, wherein message appears on the patient's mobile device concerning change in pharmacological treatment plan.

27. Telemedical system according to claim 14, **characterised in that** the module described in point f) collects data about all medical occurrences during treatment obtained both from the patient and physician, wherein this data is also provided in form of reports, in tabular and graph forms (004) (014).

28. Telemedical system according to claim 14, **characterised in that** the module described in point g) collects educational material, especially audio- and videobooks in available formats and shares them by internet, wherein physician recommends to open educational materials and sends given educational material to the patient.

29. Telemedical system according to claim 14, **characterised in that** the module described in point h) collects information about adverse events, records it and stores it on the clinical server.

30. Telemedical system according to claim 14, **characterised in that** the module described in point i) contains modules:
- for performance of cognitive trainings for patients and monitoring of changes in patients cognitive performance during treatment, and/or
- for recommending to conduct blood pressure measurement at home, and/or
- for recommending to perform rehabilitation, and/or
- for recommending to conduct PSA level measurement in the laboratory.

31. Telemedical system according to claim 30, **characterised in that** the module for conduction of cognitive trainings conducts cognitive trainings, which consist in:
- training of cognitive functions
- measuring of cognitive functions
- normalization of cognitive functions
- supervision of patients performance during cognitive trainings
- recording of cognitive functions parameters
- generation of graphic reports
- analysis of those parameters during time of therapy

32. Telemedical system according to claim 14, **characterised in that** it informs the patient about a visit in medical practice/consulting room, enables patient registration for a visit in medical practice/consulting room and generates medical documentation in electronic format.
